# EUROPEAN PATENT SPECIFICATION

(11) **EP 4 262 627 B1**
(45) Date of publication and mention of the grant of the patent: **21.10.2026**
(21) Application number: 21827435.5
(22) Date of filing: 10.12.2021
(51) Int. Cl.: A61F 2/16

(54) **INTRAOCULAR LENS WITH ROTATIONAL RESISTANCE AND NEGATIVE DYSPHOTOPSIA MITIGATION**
INTRAOKULARLINSE MIT ROTATIONSWIDERSTAND UND NEGATIVER DYSPHOTOPSIEMINDERUNG
LENTILLE INTRAOCULAIRE AVEC RÉSISTANCE À LA ROTATION ET ATTÉNUATION DE LA DYSPHOTOPSIE NÉGATIVE

(30) Priority: 17.12.2020 US 202063126760 P
(43) Date of publication of application: 25.10.2023
(73) Proprietor: Alcon Inc., 1701 Fribourg (CH)
(72) Inventor: DAS, Kamal K., Fort Worth, Texas 76134 (US); SCOTT, James M., Fort Worth, Texas 76134 (US)
(74) Representative: Murgitroyd & Company
(86) International application number: PCT/IB2021/061539
(87) International publication number: WO 2022/130141

(56) References cited:
- US-A- 6 027 531
- US-A1- 2005 021 137
- US-A1- 2016 184 088
- US-A1- 2016 317 287
- US-A1- 2020 345 481
- US-B2- 8 647 384

## Description

### TECHNICAL FIELD

The invention set forth in the appended claims relates generally to ophthalmic intraocular lenses.

### BACKGROUND

The human eye can suffer a number of maladies, which can cause mild deterioration to complete loss of vision in some instances. While contact lenses and eyeglasses can compensate for some ailments, ophthalmic surgery may be required for others. In some instances, implants may be beneficial or desirable. For example, an intraocular lens may replace a clouded natural lens within an eye to improve vision.

While the benefits of intraocular lenses and other implants are known, improvements to lenses, delivery systems, components, and processes continue to improve outcomes and benefit patients.

Reference is made to the documents US8647384, US2020/345481, US2016/317287, US6027531, US2016/184088 and US 2005/021137 A1 which have been cited as exemplary of the background state of the art. US 2005/021137 A1 relates to an aspheric, multifocal intraocular lens comprising different optical sectors, including spherical and aspheric portions, which are separated by discontinuities. These discontinuities are boundaries between surfaces of different curvature, designed to provide a continuous dioptric range for near, intermediate, and far vision. US 8,647,384 B2 discloses an accommodating IOL where a separate elastic ring is disposed around the optic sidewall.

### BRIEF SUMMARY

The scope of the invention is as defined in the appended claims. Accordingly, there is provided an intraocular lens as claimed in claim 1. Further optional features are provided in the dependent claims. Illustrative embodiments are also provided to enable a person skilled in the art to make and use the claimed subject matter.

For example, some embodiments comprise an intraocular lens that can take advantage of precise capsulorhexis openings created with femtosecond lasers to improve rotational resistance and reduce negative dysphotopsia or visual disturbances. More particularly, some embodiments may connect the anterior capsule to an intraocular lens using the capsulorhexis opening, which can increase rotational resistance of the lens and sequester the edge of the capsulorhexis opening.

More generally, an ophthalmic lens comprises a posterior optic surface and an anterior optic surface, which comprises a central optic, a peripheral optic, and a capsule rim separating the central optic and the peripheral optic. An optic edge couple the posterior optic surface to the peripheral optic. The capsule rim may be symmetric or asymmetric in various embodiments. In more particular embodiments, the capsule rim may form a surface at an angle of at least ninety (90) degrees to the peripheral optic. In some embodiments, the central optic may comprise an optic axis, and the capsule rim may form a surface that is substantially parallel to the optic axis.

The capsule rim may have a thickness that is at least as thick as the thickness of an anterior capsular bag. For example, a suitable thickness may be in a range of about 19 micrometers to about 20 micrometers. In some embodiments, the thickness of the capsule rim may be up to five (5) times the thickness of an anterior capsular bag.

Additionally, or alternatively, the central optic or the capsule rim may have a radius configured to provide elastic tension in a capsulorhexis. A radius in a range of about 2 millimeters to about 3 millimeters may be suitable for some embodiments.

Additionally, or alternatively, some embodiments of the peripheral optic may comprise a textured surface, which can be configured to reduce transmission of or diffuse the scatter light.

In some embodiments, the capsule rim may comprise a closed curve having at least one convex arc and at least one concave arc.

A method of implanting an intraocular lens into a capsular bag of an eye may comprise creating a capsulorhexis in an anterior portion of the capsular bag; removing the natural lens from the capsular bag; and inserting the intraocular lens into the capsular bag, wherein the intraocular lens comprises an anterior optic surface having a peripheral optic disposed within the capsular bag, a capsular rim adjacent to an edge of the capsulorhexis, and a central optic disposed within the capsulorhexis and substantially flush with the capsular bag. In some embodiments, the intraocular lens may comprise at least two haptics that engage the capsular bag to secure the intraocular lens. Additionally, or alternatively, the central optic may have a width larger than a width of the capsulorhexis to create tension in the capsulorhexis. Some embodiments of the peripheral optic may additionally comprise a textured surface configured to reduce transmission of or scatter light.

The method of implanting the intraocular lens does not form part of the invention.

Features, elements, and aspects described in the context of some embodiments may also be omitted, combined, or replaced by alternative features. Other features, objectives, advantages, and a preferred mode of making and using the claimed subject matter are described in greater detail below with reference to the accompanying drawings of illustrative embodiments.

### BRIEF DESCRIPTION OF THE DRAWINGS

The accompanying drawings illustrate some objectives, advantages, and a preferred mode of making and using some embodiments of the claimed subject matter. Like reference numbers represent like parts in the examples.
Figure 1 is a top view of an example of a lens, illustrating various features that may be associated with some embodiments.
Figure 2 is a side view of the lens of Figure 1.
Figure 3 is a detail view of the lens of Figure 2.
Figure 4 is a top view of another example of a lens, illustrating various features that may be associated with some embodiments.
Figure 5 is a side view of the lens of Figure 4.
Figure 6 is an isometric view of the lens of Figure 4.
Figure 7 is a top view of another example of a lens, illustrating additional or alternative details that may be associated with some embodiments.
Figure 8 is a side view of the lens of Figure 7.
Figure 9 is a detail view of a portion of the lens of Figure 8.
Figure 10 is a top view of another example of a lens, illustrating additional details that may be associated with some embodiments.
Figures 11A-11B are schematic diagrams illustrating an example use of the lens of Figure 1.
Figure 12 is a detail view of Figure 11B.

### DESCRIPTION OF EXAMPLE EMBODIMENTS

The following description of example embodiments provides information that enables a person skilled in the art to make and use the subject matter set forth in the appended claims, but it may omit certain details already well known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive an implant. However, as should be recognized by those skilled in the art, this frame of reference is merely a descriptive expedient rather than a strict prescription.

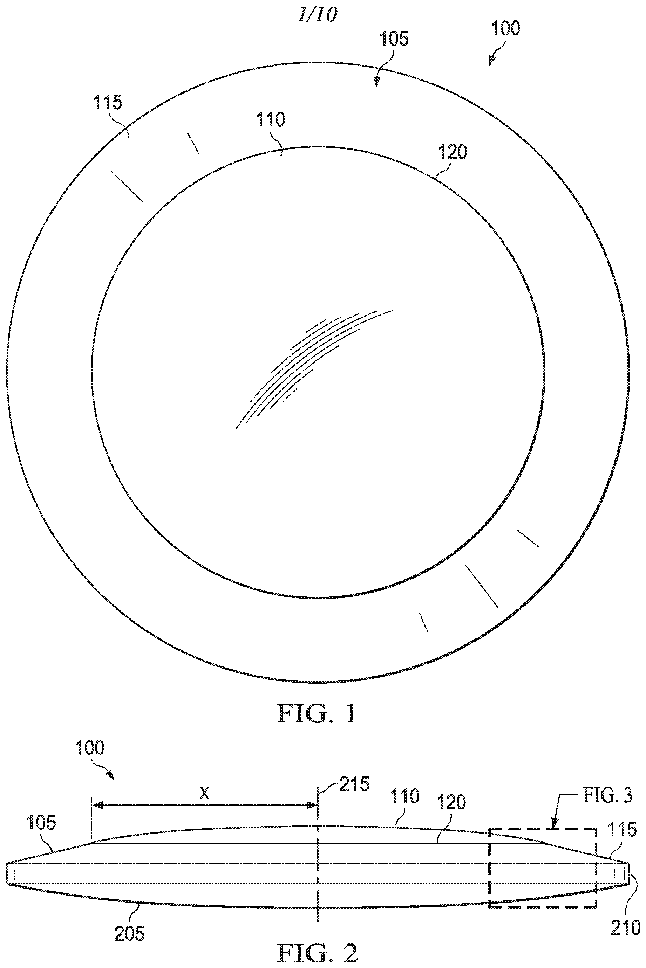

Figure 1 is a top view of an example of a lens 100 illustrating various features that may be associated with some embodiments. As illustrated in the example of Figure 1, the lens 100 may include an anterior optic surface 105, which may comprise a central optic 110, a peripheral optic 115, and a capsule rim 120 separating the central optic 110 and the peripheral optic 115. In some embodiments, the capsule rim 120 may define at least a portion of the central optic 110. Additionally, or alternatively, some embodiments of the capsule rim 120 may be symmetric, as illustrated in Figure 1. More specifically, some embodiments of the capsule rim 120 may be circular.

Figure 2 is a side view of the lens 100 of Figure 1, illustrating additional features that may be associated with some embodiments. For example, the lens 100 of Figure 2 includes a posterior optic surface 205 and an optic edge 210 coupling the posterior optic surface 205 to the anterior optic surface 105. More particularly, as illustrated in Figure 2, the optic edge 210 may couple the posterior optic surface 205 to the peripheral optic 115 of the anterior optic surface 105. Additionally, Figure 2 further illustrates the circular example of the capsule rim 120 of Figure 1 having a radius X about an optical axis 215. A radius X in a range of about 2 millimeters to about 3 millimeters may be suitable for some embodiments. Moreover, as illustrated in Figure 2, the optical axis 215 may be substantially orthogonal to the central optic 110 in some embodiments.

The optic edge 210 of Figure 2 is a straight edge. In other examples, the optic edge 210 may be a curved edge or may comprise more than one curved edge. For example, the optic edge 210 may include a plurality of distinct curved surfaces, which may be tangentially connected to one another. In some embodiments, each of the curved surfaces may have a different radius of curvature. Additionally, the peripheral optic 115 may be tangentially connected to the optic edge 210 in some examples.

The anterior optic surface 105 and the posterior optic surface 205 of Figure 2 are both convex. In other embodiments, the anterior optic surface 105 and the posterior optic surface 205 may have any suitable combination of concave, convex, or flat surface profiles. The anterior optic surface 105, the posterior optic surface 205, or both may include any suitable spherical, aspheric, refractive, diffractive, or phase shifting features to focus and direct light.

In some examples, the lens 100 may be an intraocular lens, and the anterior optic surface 105 may have an anterior optical diameter in a range of 4.5 millimeters to 7 millimeters. The thickness of the optic edge 210 (measured along the optical axis 215 of the lens 100) may be in a range of 0.15 millimeters and 0.45 millimeters or higher. The posterior optic surface 205 can have a posterior optical diameter in a range of 5.0 millimeters to 8.5 millimeters. The posterior optical diameter can be dependent on the anterior optical diameter, and in some embodiments, the posterior optical diameter may be greater than the anterior optical diameter.

The anterior optical diameter and the posterior optical diameter may be selected based on the size of the eye or pupil of a patient. For example, visual disturbances such as positive and negative dysphotopsia can occur more frequently in patients with larger pupil sizes. A relatively larger anterior optical diameter and posterior optical diameter can reduce or eliminate glare and other visual disturbances by allowing the lens 100 to receive and focus greater amounts of light to the retina.

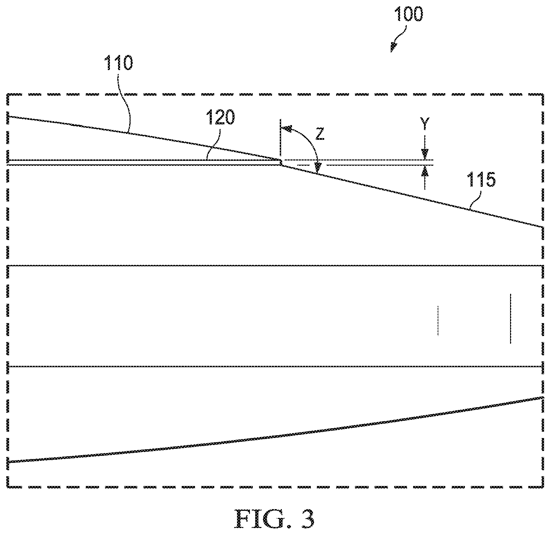

Figure 3 is a detail view of the lens 100 of Figure 2, illustrating additional features that may be associated with some embodiments. In the example of Figure 3, the capsule rim 120 has an edge-to-edge thickness Y. The thickness Y may vary, but a thickness Y in a range of about 19 micrometers to about 20 micrometers may be suitable for many applications. For example, the thickness Y may be about 19.5 micrometers in some embodiments. A capsule rim 120 of suitable dimensions may be manufactured by modifying the anterior optic surface 105 to add a step down from the central optic 110 with lathing or by incorporating the features in molded lenses, for example. Additionally, the capsule rim 120 of Figure 3 forms a surface at an angle Z relative to the peripheral optic 115. An angle Z of at least 90 degrees may be advantageous for some embodiments. Additionally, or alternatively, the capsule rim 120 may form a surface that is substantially parallel to the optical axis 215. In other embodiments, the central optic 110 may be recessed relative to the peripheral optic 115, and the capsule rim 120 may provide a step down from the peripheral optic 115 to the central optic 110.

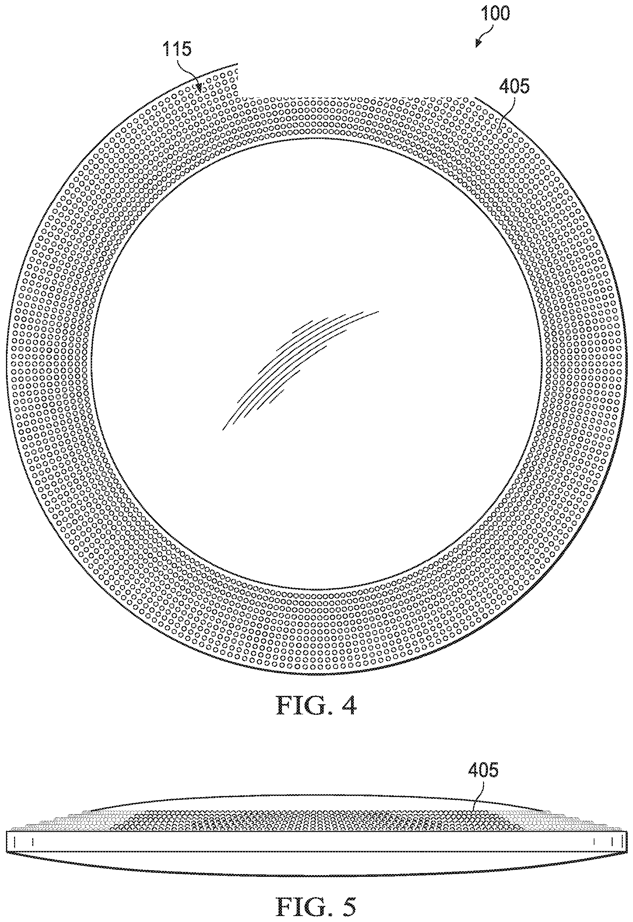

Figure 4 is a top view of another example of the lens 100, illustrating additional or alternative details that may be associated with some embodiments. As illustrated in the example of Figure 4, the peripheral optic 115 may have a textured surface 405 in some embodiments. The texture surface 405 may comprise or consist essentially of a pattern, which in some examples may be transferred from tooling or from injection molded wafers. In some examples, the textured surface 405 may be directly machined into the lens 100 and/or etched into the lens 100 during known manufacturing processes. Subsequently, the textured surface 405 may range from less than a nanometer to hundreds of microns in depth.

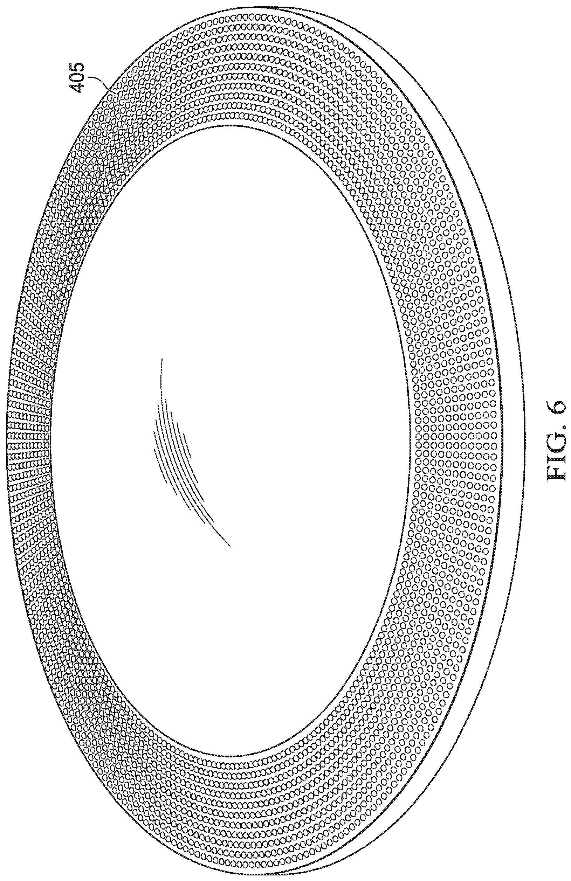

Figure 5 is a side view of the lens 100 of Figure 4, and Figure 6 is an isometric view of the lens 100 of Figure 4 illustrating the textured surface 405 in additional detail.

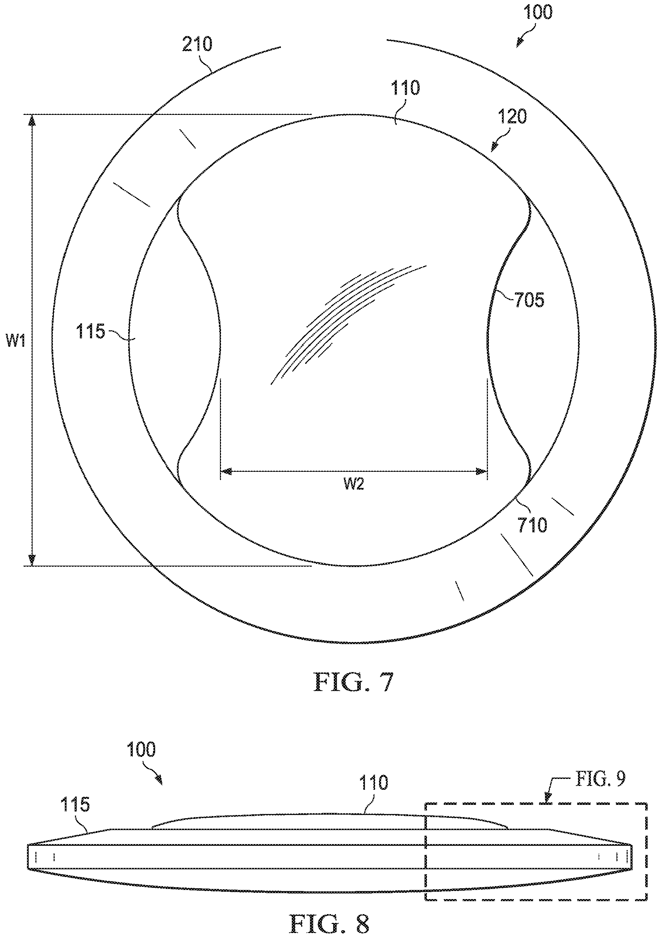

Figure 7 is a top view of another example of the lens 100, illustrating additional or alternative details that may be associated with some embodiments. For example, some embodiments of the capsule rim 120 may have an alternative profile. Some embodiments of the capsule rim 120 may be a closed curve having at least one convex arc and one concave arc. In the example of Figure 7, the capsule rim 120 has two concave arcs 705 and two convex arcs 710, which are oriented to maintain symmetry in the profile of the capsule rim 120. The convex arcs 710 may be concentric with the optic edge 210 in some examples, as illustrated in Figure 7. The capsule rim 120 of Figure 7 may additionally or alternatively be characterized by an outer width W1 between the two convex arcs 710 and an inner width W2 between the two concave arcs 705. In other examples, the capsule rim 120 may have an asymmetrical profile. The width W1 and the width W2 may vary according to anatomical or procedural requirements. For example, a width W1 in a range of about 3 millimeters to about 6.5 millimeters or approximately 0.5 millimeters less than the optical diameter, and more particularly about 5.5 millimeters for a 6-millimeter optical body, may be suitable for many applications. The width W2 is generally less than the width W1. A ratio of W2/W1 in a range of about 1 to about 0.5 may be suitable for some examples.

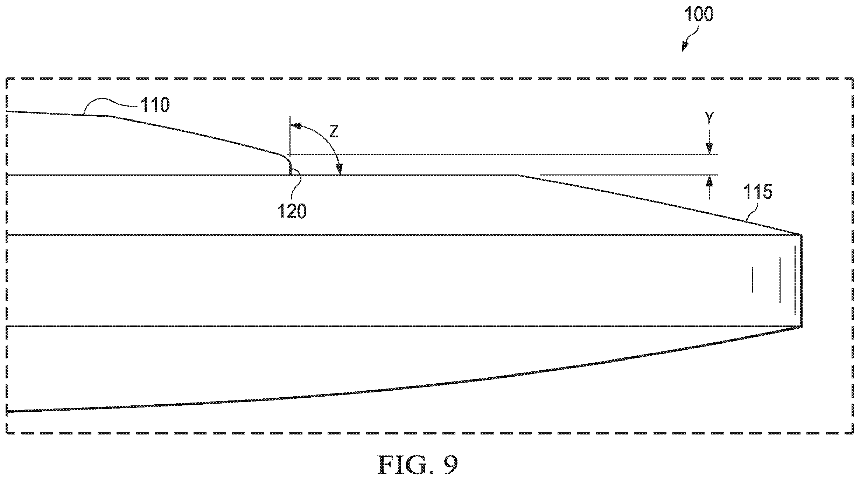

Figure 8 is a side view of the lens 100 of Figure 7, and Figure 9 is a detail view of a portion of the lens 100 of Figure 8 illustrating additional details that may be associated with some embodiments. For example, at least a portion of the capsule rim 120 of Figure 9 is curved. In some embodiments, the capsule rim 120 may include a plurality of distinct curved surfaces, which may be tangentially connected to one another. In some embodiments, each of the curved surfaces may have a different radius of curvature. Additionally, the capsule rim 120 may be tangentially connected to the central optic 110, the peripheral optic 115, or both in some examples.

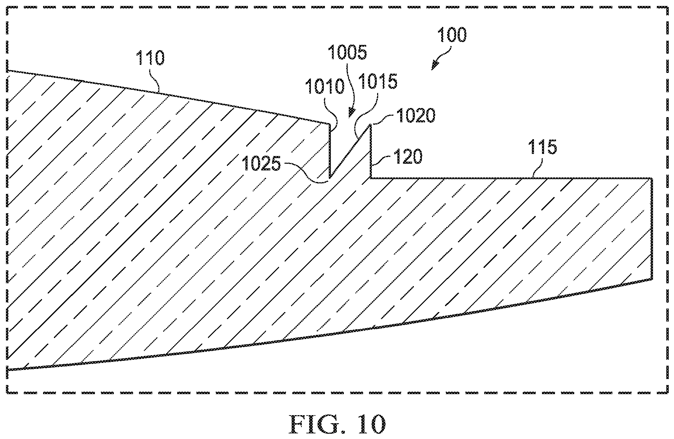

Figure 10 is a detail view of another example of the lens 100, illustrating additional or alternative details that may be associated with some embodiments. For example, as illustrated in Figure 10, some embodiments of the lens 100 may additionally comprise a transition band 1005 for diffusing or redirecting light away from the capsule rim 120. The transition band 1005 may be disposed inboard of the capsule rim 120. As illustrated in Figure 10, for example, the transition band 1005 may be disposed between the central optic 110 and the capsule rim 120. In some embodiments, the transition band 1005 may comprise or consist essentially of a transition rim 1010 and a bridge 1015. The transition rim 1010 may be parallel to the capsule rim 120, and the bridge 1015 may connect an anterior edge 1020 of the capsule rim 120 to a posterior edge 1025 of the transition rim 1010. In some examples, the transition rim 1010 and the bridge 1015 may be flat surfaces, as illustrated in Figure 10; in other examples, the transition rim 1010, the bridge 1015, or both may be curved. For example, the transition rim 1010, the bridge 1015, or both may be convex, concave, or a combination of convex surfaces, convex surfaces, or both. Additionally, or alternatively, the capsule rim 120 may provide a step down from the peripheral optic 115 in some embodiments, and one or more of the central optic 110, the transition rim 1010, and the bridge 1015 may be at least partially recessed relative to the peripheral optic 115.

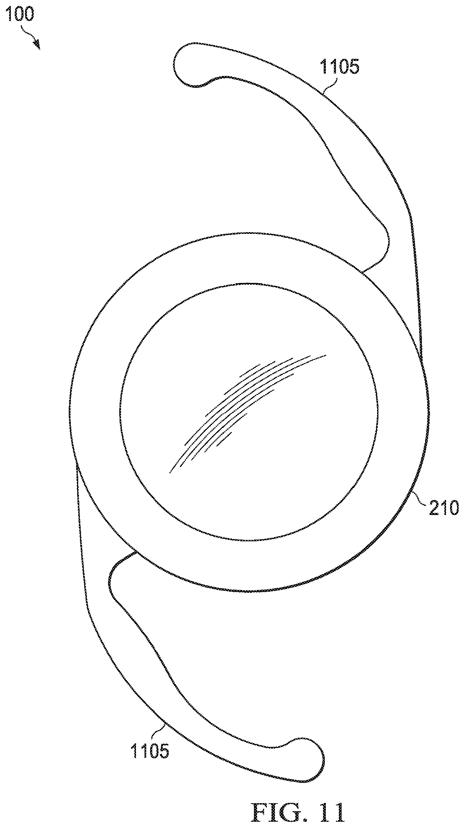

Figure 11 is a top view of another example of the lens 100 illustrating additional details that may be associated with some embodiments. For example, some embodiments may additionally comprise one or more haptics 1105, which may be coupled to the optic edge 210.

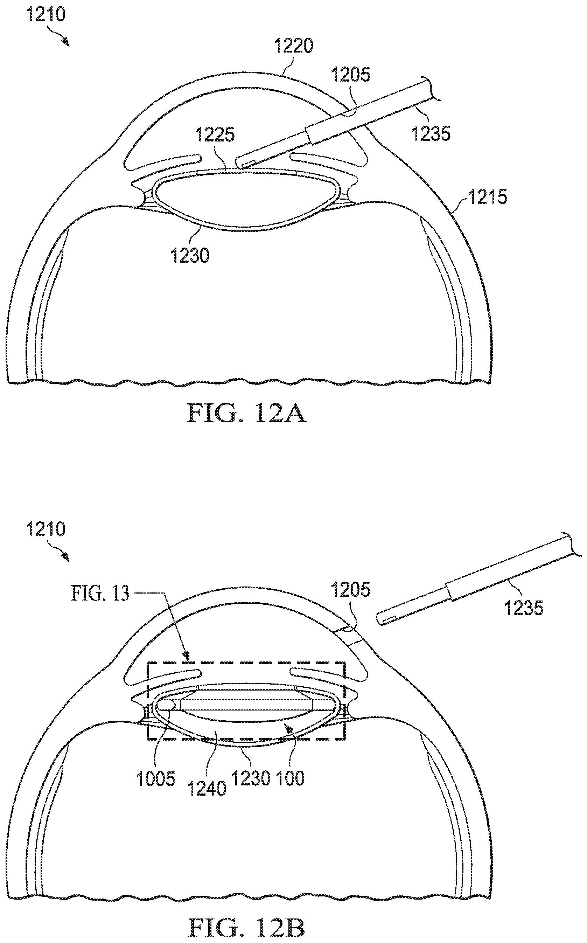

Figures 12A-12B are schematic diagrams further illustrating an example use of the lens 100. As illustrated, an incision 1205 may be made in an eye 1210 by a surgeon, for example. In some instances, the incision 1205 may be made through the sclera 1215 of the eye 1210. In other instances, an incision may be formed in the cornea 1220 of the eye 1210. A capsulorhexis opening 1225 can be made in an anterior portion of the capsular bag 1230. For example, a femtosecond laser can be used to create the capsulorhexis opening 1225 with very precise dimensions. The capsulorhexis radius for an average eye size may be about 2.25 millimeters. The incision 1205 may be sized to permit removal of the natural lens from the capsular bag 1230. A nozzle 1235 may also be at least partially inserted through the incision 1205 to deliver the lens 100 into the capsular bag 1230. For example, in some instances, the size of the incision 1205 may have a length less than about 3000 microns (3 millimeters). In other instances, the incision 1205 may have a length of from about 1000 microns to about 1500 microns, from about 1500 microns to about 2000 microns, from about 2000 microns to about 2500 microns, or from about 2500 microns to about 3000 microns.

The nozzle 1235 can be inserted through the incision 1205 into an interior portion 1240 of the capsular bag 1230. The lens 100 can then be ejected through the nozzle 1235 into the capsular bag 1230. In some applications, the lens 100 may be delivered in a folded configuration and can revert to an initial, unfolded state, within the capsular bag 1230, as shown in Figure 12B. The capsular bag 1230 can retain the lens 100 within the eye 1210 in a relationship relative to the eye 1210 so that the lens 100 refracts light directed to the retina (not shown). In some embodiments, haptics, such as the haptics 1105 can engage the capsular bag 1230 to secure the lens 100 therein.

After dispensing the lens 100 into the capsular bag 1230, the nozzle 1235 may be removed from the eye 1210 through the incision 1205, and the eye 1210 can be allowed to heal over a period of time.

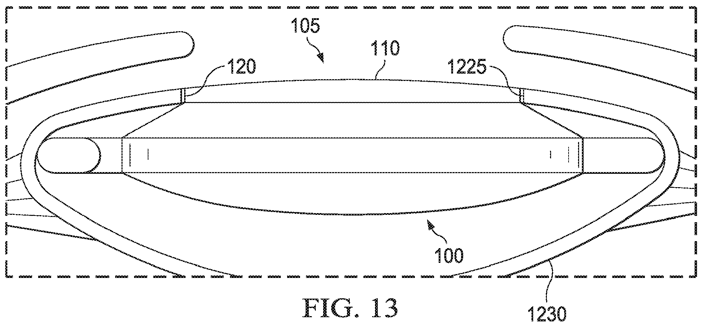

Figure 13 is a detail view of Figure 12B, illustrating additional details of the lens 100 disposed within the capsular bag 1230. In the example of Figure 13, the capsule rim 120 is configured to sequester the capsulorhexis opening 1225. For example, in Figure 13 the capsule rim 120 is disposed adjacent to an edge of the capsulorhexis opening 1225, and the central optic 110 is disposed within the capsulorhexis opening 1225. The precision of the capsulorhexis opening 1225 created by a femtosecond laser can allow the capsule rim 120 to interact with the edges of the capsulorhexis opening 1225.

As shown in the example of Figure 13, the central optic 110 may be positioned to be substantially flush with the outer surface of the capsular bag 1230, which can substantially reduce or eliminate a step change or other discontinuity between the capsular bag 1230 and the anterior optic surface 105. As illustrated in Figure 13, the capsular bag 1230 may not overlap any portion of the central optic 110. In some embodiments, the central optic 110 may be disposed posterior to the outer surface of the capsular bag 1230, which can redirect large, off-axis light and further mitigate glare and dysphotopsia effects. The peripheral optic 115 can be recessed within the capsular bag 1230. In some embodiments, the peripheral optic 115 may interact with the interior surface of the capsular bag 1230.

In some embodiments, the thickness Y (see Figure 3) of the capsule rim 120 may be substantially equal to the thickness of the edge of the capsulorhexis opening 1225, as illustrated in the example of Figure 13. In other examples, the thickness Y may be up to about five (5) times the thickness of the edge of the capsulorhexis opening 1225, so that the central optic 110 remains substantially flush with the outer surface of the capsular bag 1230 and the capsule rim 120 may extend posterior to the capsulorhexis opening 1225.

In some embodiments the radius X (see Figure 2) of the capsule rim 120 may be slightly larger than the radius of the capsulorhexis opening 1225 to provide elastic tension. For example, if the capsulorhexis opening 1225 has a radius of 2.25 millimeters, the capsule rim 120 may have a radius of about 2.5 millimeters. In other examples, the capsule rim 120 may not be symmetric and may provide elastic tension along one or more selected axis. For example, some embodiments of the capsule rim 120 may provide elastic tension orthogonal to the haptics.

Additionally, or alternatively, some embodiments may have the textured surface 405 that can scatter transient light from the periphery and edge of the lens 100.

The systems, apparatuses, and methods described herein may provide significant advantages. For example, the lens 100 can provide more rotational resistance than other lenses, including open-loop, single piece designs, which may be particularly advantageous for toric lenses where each degree of rotation can cause significant loss in cylinder power. Some embodiments of the lens 100 provide significant rotational stability and may substantially eliminate lens rotation until the capsular bag fuses. Additionally, features such as the capsule rim 120 can sequester the edges of the capsulorhexis opening, which, in contrast to other designs, can prevent the anterior capsule from covering the central optic. Such features can substantially reduce or eliminate reflections from the capsule, which can substantially reduce or eliminate the incidence of negative dysphotopsia resulting from light entering the eye at very large visual angles and striking the edge of the capsulorhexis opening 1225.

While shown in a few illustrative embodiments, a person having ordinary skill in the art will recognize that the systems, apparatuses, and methods described herein are susceptible to various changes and modifications that fall within the scope of the appended claims. For example, various features such as the capsule rim 120 and the textured surface 405 can be incorporated into plate or multi-piece lenses of hydrophobic or hydrophilic materials. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations, features of the capsule rim 120 and the textured surface 405 can be combined in various ways.

The claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described in the context of some embodiments may also be omitted, combined, or replaced by alternative features serving the same, equivalent, or similar purpose without departing from the scope of the invention defined by the appended claims.

## Claims

1. An intraocular ophthalmic lens, IOL, (100) comprising:
a posterior optic surface (205);
an anterior optic surface (105) comprising a central optic (110), a peripheral optic (115), and a capsule rim (120) separating the central optic (110) and the peripheral optic (115); and
an optic edge (210) coupling the posterior optic surface (205) to the peripheral optic (115);
wherein the capsule rim (120) is configured to sequester an edge of a capsulorhexis opening, when the lens (100) is located within a capsular bag such that the capsule rim (120) is disposed adjacent to the edge of the capsulorhexis opening, and the central optic is disposed within the capsulorhexis opening.

2. The ophthalmic lens of claim 1, wherein the capsule rim (120) is symmetric.

3. The ophthalmic lens of claim 1, wherein the capsule rim (120) is asymmetric.

4. The ophthalmic lens of any preceding claim wherein the capsule rim (120) forms a surface at an angle of at least 90 degrees to the peripheral optic (115).

5. The ophthalmic lens of any preceding claim, wherein the central optic (110) comprises an optic axis and the capsule rim (120) forms a surface that is substantially parallel to the optic axis.

6. The ophthalmic lens of any preceding claim, wherein the capsule rim (120) has a thickness in a range of about 19 micrometers to about 20 micrometers.

7. The ophthalmic lens of any preceding claim, wherein the capsule rim (120) has a thickness substantially equal to an anatomical thickness of an anterior capsular bag.

8. The ophthalmic lens of any preceding claim, wherein the capsule rim (120) has a radius in a range of about 2 millimeters to about 3 millimeters.

9. The ophthalmic lens of any preceding claim, wherein the peripheral optic (115) comprises a textured surface (405).

10. The ophthalmic lens of claim 9, wherein the textured surface (405) is configured to diffuse light.

11. The ophthalmic lens of any preceding claim, further comprising a transition band (1005), comprising one or more surfaces located between the central optic and capsule rim, and configured to diffuse or redirect light.

12. The ophthalmic lens of any preceding claim, wherein the central optic (110) is recessed relative to the peripheral optic (115).

13. The ophthalmic lens of claim 1, wherein the capsule rim (120) comprises a closed curve having at least one convex arc (710) and at least one concave arc (705).

14. The ophthalmic lens of claim 13, wherein the capsule rim (120) is symmetric.

15. The ophthalmic lens of claim 13, wherein the capsule rim (120) is asymmetric.

## Patentansprüche

1. Ophthalmische Intraokularlinse, IOL, (100), umfassend:
eine posteriore optische Oberfläche (205);
eine anteriore optische Oberfläche (105), die eine zentrale Optik (110), eine periphere Optik (115) und einen Kapselrand (120) umfasst, der die zentrale Optik (110) und die periphere Optik (115) trennt; und
eine Optikkante (210), welche die posteriore optische Oberfläche (205) mit der peripheren Optik (115) koppelt;
wobei der Kapselrand (120) dazu ausgelegt ist, eine Kante einer Kapsulorhexis-Öffnung zu sequestrieren, wenn sich die Linse (100) innerhalb eines Kapselsacks befindet, so dass der Kapselrand (120) benachbart zu der Kante der Kapsulorhexis-Öffnung angeordnet ist, und die zentrale Optik innerhalb der Kapsulorhexis-Öffnung angeordnet ist.

2. Ophthalmische Linse nach Anspruch 1, wobei der Kapselrand (120) symmetrisch ist.

3. Ophthalmische Linse nach Anspruch 1, wobei der Kapselrand (120) asymmetrisch ist.

4. Ophthalmische Linse nach einem der vorhergehenden Ansprüche, wobei der Kapselrand (120) eine Oberfläche in einem Winkel von mindestens 90 Grad zu der peripheren Optik (115) bildet.

5. Ophthalmische Linse nach einem der vorhergehenden Ansprüche, wobei die zentrale Optik (110) eine optische Achse umfasst und der Kapselrand (120) eine Oberfläche bildet, die im Wesentlichen parallel zu der optischen Achse ist.

6. Ophthalmische Linse nach einem der vorhergehenden Ansprüche, wobei der Kapselrand (120) eine Dicke in einem Bereich von etwa 19 Mikrometer bis etwa 20 Mikrometer aufweist.

7. Ophthalmische Linse nach einem der vorhergehenden Ansprüche, wobei der Kapselrand (120) eine Dicke aufweist, die im Wesentlichen gleich einer anatomischen Dicke eines anterioren Kapselsacks ist.

8. Ophthalmische Linse nach einem der vorhergehenden Ansprüche, wobei der Kapselrand (120) einen Radius in einem Bereich von etwa 2 Millimeter bis etwa 3 Millimeter aufweist.

9. Ophthalmische Linse nach einem der vorhergehenden Ansprüche, wobei die periphere Optik (115) eine texturierte Oberfläche (405) umfasst.

10. Ophthalmische Linse nach Anspruch 9, wobei die texturierte Oberfläche (405) dazu ausgelegt ist, Licht zu diffundieren.

11. Ophthalmische Linse nach einem der vorhergehenden Ansprüche, ferner umfassend ein Übergangsband (1005), das eine oder mehrere Oberflächen umfasst, die sich zwischen der zentralen Optik und dem Kapselrand befinden und dazu ausgelegt sind, Licht zu diffundieren oder umzulenken.

12. Ophthalmische Linse nach einem der vorhergehenden Ansprüche, wobei die zentrale Optik (110) relativ zu der peripheren Optik (115) ausgespart ist.

13. Ophthalmische Linse nach Anspruch 1, wobei der Kapselrand (120) eine geschlossene Kurve mit mindestens einem konvexen Bogen (710) und mindestens einem konkaven Bogen (705) umfasst.

14. Ophthalmische Linse nach Anspruch 13, wobei der Kapselrand (120) symmetrisch ist.

15. Ophthalmische Linse nach Anspruch 13, wobei der Kapselrand (120) asymmetrisch ist.

## Revendications

1. Lentille intraoculaire ophtalmique, LIO (100), comprenant :
une surface optique postérieure (205) ;
une surface optique antérieure (105) comprenant une optique centrale (110), une optique périphérique (115) et un rebord de capsule (120) séparant l'optique centrale (110) et l'optique périphérique (115) ; et
un bord optique (210) couplant la surface optique postérieure (205) à l'optique périphérique (115) ;
le rebord de capsule (120) étant configuré pour séquestrer un bord d'une ouverture de capsulorhexis, lorsque la lentille (100) est située dans un sac capsulaire de telle sorte que le rebord de capsule (120) est disposé de manière adjacente au bord de l'ouverture de capsulorhexis, et que l'optique centrale est disposée dans l'ouverture de capsulorhexis.

2. Lentille ophtalmique selon la revendication 1, le rebord de capsule (120) étant symétrique.

3. Lentille ophtalmique selon la revendication 1, le rebord de capsule (120) étant asymétrique.

4. Lentille ophtalmique selon l'une quelconque des revendications précédentes, le rebord de capsule (120) formant une surface formant un angle d'au moins 90 degrés par rapport à l'optique périphérique (115).

5. Lentille ophtalmique selon l'une quelconque des revendications précédentes, l'optique centrale (110) comprenant un axe optique et le rebord de capsule (120) formant une surface qui est sensiblement parallèle à l'axe optique.

6. Lentille ophtalmique selon l'une quelconque des revendications précédentes, le rebord de capsule (120) ayant une épaisseur dans une plage d'environ 19 micromètres à environ 20 micromètres.

7. Lentille ophtalmique selon l'une quelconque des revendications précédentes, le rebord de capsule (120) ayant une épaisseur sensiblement égale à une épaisseur anatomique d'un sac capsulaire antérieur.

8. Lentille ophtalmique selon l'une quelconque des revendications précédentes, le rebord de capsule (120) ayant un rayon dans une plage d'environ 2 millimètres à environ 3 millimètres.

9. Lentille ophtalmique selon l'une quelconque des revendications précédentes, l'optique périphérique (115) comprenant une surface texturée (405).

10. Lentille ophtalmique selon la revendication 9, la surface texturée (405) étant configurée pour diffuser la lumière.

11. Lentille ophtalmique selon l'une quelconque des revendications précédentes, comprenant en outre une bande de transition (1005), comprenant une ou plusieurs surfaces situées entre l'optique centrale et le rebord de capsule, et configurées pour diffuser ou rediriger la lumière.

12. Lentille ophtalmique selon l'une quelconque des revendications précédentes, l'optique centrale (110) étant en retrait par rapport à l'optique périphérique (115).

13. Lentille ophtalmique selon la revendication 1, le rebord de capsule (120) comprenant une courbe fermée ayant au moins un arc convexe (710) et au moins un arc concave (705).

14. Lentille ophtalmique selon la revendication 13, le rebord de capsule (120) étant symétrique.

15. Lentille ophtalmique selon la revendication 13, le rebord de capsule (120) étant asymétrique.
